# EUROPEAN PATENT SPECIFICATION

(11) **EP 4 510 993 B1**
(45) Date of publication and mention of the grant of the patent: **26.08.2026**
(21) Application number: 23724913.1
(22) Date of filing: 19.04.2023
(51) Int. Cl.: A61K 8/04, A61K 8/31, A61K 8/33, A61K 8/37, A61Q 9/04, A61K 8/92

(54) **HAIR REMOVAL COMPOSITION DISPENSABLE FROM A SPRAY CAN**
AUS EINER SPRÜHDOSE SPENDEBARE HAARENTFERNUNGSZUSAMMENSETZUNG
COMPOSITION D'ÉPILATION POUVANT ÊTRE DISTRIBUÉE À PARTIR D'UNE BOMBE AÉROSOL

(30) Priority: 20.04.2022 IT 202200007823; 03.04.2023 US 202363456715 P
(43) Date of publication of application: 26.02.2025
(73) Proprietor: Romani, Paolo, 61122 PESARO (PU) (IT)
(72) Inventor: Romani, Paolo, 61122 PESARO (PU) (IT)
(74) Representative: Studio Torta S.p.A.
(86) International application number: PCT/IB2023/054001
(87) International publication number: WO 2023/203504

(56) References cited:
- GB-A- 2 337 460
- JP-A- H08 283 131

## Description

### Cross-Reference to Related Applications

This patent application is related to Italian Patent Application No. 102022000007823 filed on April 20, 2022, and to US Provisional Application no. 63/456,715 filed on April 3, 2023, the entire disclosure of which is incorporated herein by reference.

### Technical Field

The present invention relates to a hair removal composition and a use of such a composition. The present invention also relates to a hair removal product (containing the mentioned composition) and a kit comprising said product.

### Background of the Invention

In the field of hair removal it is known to use a wax that is heated so that it assumes a fluid consistency and can be applied, still hot, on an area of the skin to be treated. At this point, a layer of nonwoven fabric is made to adhere onto it with a little pressure. Once re-solidified, the wax is removed by pulling the nonwoven fabric swiftly in the opposite direction to the hair growth so that, while it is being pulled off, it removes the same hair (by grabbing it therewith).

This procedure, being relatively complex (mainly due to the need to heat the wax), is rarely used in the domestic environment and has some further drawbacks, including the fact that, when applied hot, it can create, to predisposed subjects, rashes on the skin.

In the domestic environment, therefore, so-called depilatory strips are more often used, i.e. support strips (made of paper, plastic and/or fabric) on the surface of which a layer of a cold active wax is arranged. Typically, these strips are marketed coupled i.e. like two support strips connected by an intermediate layer of wax. When it is wished to use the strips they are separated and the respective layer of glue remains on the surface of each one.

Although the depilatory strips are easier to use, they are also not free of drawbacks: it happens with a certain frequency that the wax dries (even only partially) and becomes ineffective; during the separation phase, it is not uncommon for one or both strips to break or for the resin to distribute unevenly between the two strips.

JPH08283131 discloses a composition obtained by blending resins, turpentines and dimethyl ether/ liquefied petroleum gas in a pressure resistant can equipped with a spray mechanism for an aerosol.

Aim of the present invention is to provide a hair removal composition, a use of such composition, a hair removal product and a kit comprising such a product, which allow to overcome, at least partially, the drawbacks of the prior art and are, at the same time, of easy and economical realization and/or use.

### Summary

According to the present invention there is provided a hair removal composition, a use of such a composition, a hair removal product and a kit comprising such a product as recited in the independent claims which follow and, preferably, in any one of the claims directly or indirectly dependent on the independent claims.

### Brief Description of the Drawings

The invention is described below with reference to the accompanying figures, which show non-limiting embodiments thereof, wherein:
- Figures 1 to 4 show an efficacy proof of an embodiment of the composition in accordance with the present invention relative to depilatory strips currently on the market;
- Figure 5 is a perspective view of a hair removal product in accordance with the present invention;
- Figure 6 is a section of a part of the product of Figure 5;
- Figure 7 is a section of a further part of the product of Figure 5;
- Figures 8 to 10 show a test of efficacy of a comparison product; and
- Figures 11 to 13 show a test of the efficacy of an embodiment of the composition in accordance with the present invention;
- Figures 14 to 16 show a test of efficacy of an embodiment of the composition in accordance with the present invention; and
- Figures 17 to 19 show a test of efficacy of an embodiment of the composition in accordance with the present invention.

### Detailed Description

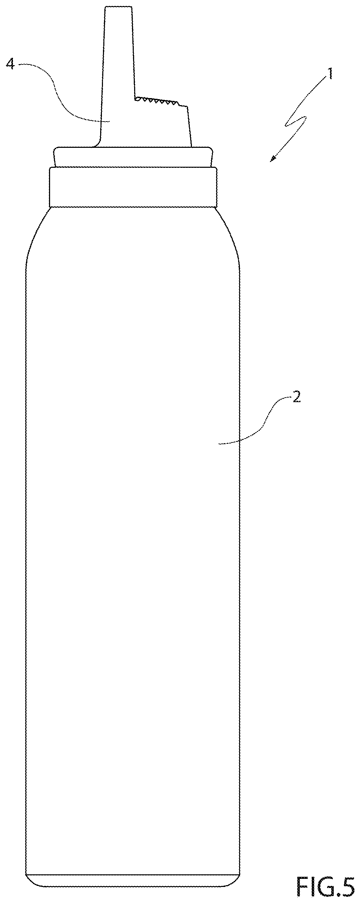

In accordance with a first aspect of the present invention, there is provided a pulling-off composition. The composition, which, in particular, is dispensable from a spray can (see Figures 5-7 wherein the can, also referred to as a hair removal product, is identified by reference numeral 1), comprises at least about 45% (in particular, at least about 50%; more in particular, at least about 55%; still more in particular, at least about 70%) by weight, relative to the total weight of the composition, of a wax; and from about 20% (in particular, from about 25%) to about 45% (in particular, to about 40%) by weight, relative to the total weight of the composition, of a propellant, which is gaseous at a temperature of 25°C and at a pressure of 1 bar and liquid at a temperature of 25°C and at a pressure of 2.8 bar (1 bar corresponds to 100000 Pa). The propellant comprises (in particular, consists of) at least one organic compound chosen from the group consisting of: C₁-C₄ aliphatic compounds (in particular, alkyls), C₂-C₆ ethers and a combination thereof (i.e. mixture).

It has been experimentally observed that a composition of this type allows to obtain a hair removal of comparable quality, even better than the known techniques. At the same time, the mentioned composition is easy to use and reduces the risk of waste.

In this regard, it has been noted that the propellant, in addition to allowing a correct dispensing of the composition (from the spray can 1) keeps the wax liquid during application (thus favouring the usability thereof) and, by evaporating (at least partially), allows the wax to (re)solidify.

Unless explicitly stated otherwise, in the present text, "Cₓ-C_{y}" refers to a group and/or a compound which is understood as having x to y carbon atoms.

Unless explicitly stated otherwise, "aliphatic" in this text means a non-aromatic and unsubstituted (unless otherwise specified), saturated or unsaturated, linear, branched and/or cyclic hydrocarbon. Non-limiting examples of aliphatic groups are: t-butyl, ethenyl, 1- or 2-propenyl, cyclohexyl.

Unless explicitly stated otherwise, by "alkyl" or "alkyl-" it is meant a saturated aliphatic (i.e. an aliphatic group without double or triple carbon-carbon bonds). Non-limiting examples of alkyls are: methyl, n-propyl, t-butyl, cyclohexyl.

By pulling-off hair removal is meant the type of hair removal that involves making a product to adhere to an area of the body (so that the product sticks to the hair) and then removing it by swiftly pulling it so that, while it is being pulled off, it removes the hair by grabbing it therewith.

Advantageously but not necessarily, by wax is meant a substance substantially solid at a temperature lower than about 40°C (in particular, lower than about 60°C; more in particular lower than about 65°C) at a pressure of 1 bar. In addition or alternatively, the wax is a substance (the substance is) substantially liquid at the temperature of about 100°C (in particular, at the temperature of about 90°C) (in particular, at a 1 bar pressure).

According to some non-limiting embodiments, the wax has a softening point (at 1 bar) greater than about 40°C (in particular, greater than about 70°C; in particular, at about 80°C) . Alternatively or additionally, the wax has a softening point (at 1 bar) lower than about 110°C (in particular, lower than about 100°C).

According to some non-limiting embodiments, the wax comprises (in particular, is) a resin (in particular, substantially liquid at the temperature of about 100°C - at a 1 bar pressure). In some specific non-limiting cases, the resin has a softening point (at 1 bar) ranging from about 70°C (in particular, about 80°C; more particularly, about 85°C) to about 99°C (in particular, to about 95°C; more in particular, to about 90°C).

The following procedure was followed to measure the softening point of a material (e.g. a wax and/or a resin). A standard-sized ring is hot-filled with resin, which is then cooled. The full ring is placed on a support inside a thermostatic oil bath. Above the full ring there is placed a steel ball of standardized size. Heating begins. The resin slowly softens and no longer resists the weight of the ball that pushes the resin downwards. When the ball touches the base of the support, the temperature is read.

More precisely, to measure the softening point of a material (e.g. a wax and/or a resin), the provisions of ASTM E28 regulation (July 2018 edition) were followed.

Typically, moreover, the wax is substantially insoluble in water and soluble in an organic solvent.

Advantageously but not necessarily, the propellant is gaseous also at a temperature of about 0°C (in particular, also at a temperature of about -20°C) and at a pressure of 1 bar. Alternatively or additionally, the propellant is substantially liquid at a temperature of about 25°C and also at a pressure of about 2.5 bar (in particular, also at a pressure of about 2 bar).

According to some non-limiting embodiments, the propellant has a boiling point between about -45°C (in particular, about -30°C; more in particular, about -25°C) and 0°C (in particular, about -10°C).

To measure the boiling point of a substance (such as of the propellant), it is proceeded in this way. In a test tube, one pours 15-20 ml of the liquid substance to be analysed and a glass capillary (with an inner diameter of about 1 mm and a wall thickness of about 0.1-0.2 mm) open at an overturned end (i.e. with the closed part turned upwards, and the open part drawing in the substance). At this point, an air bubble occupying most of the capillary so positioned inside a test tube is observed. With a clamp, the thermometer is positioned at the same height as the substance contained in the tube and the whole is immersed in a beaker containing a further liquid. It is made sure that the level of the liquid is higher than the level of the substance to be analysed. It is heated and during heating bubbles escaping from the mouth of the overturned capillary can be observed. As soon as the rapid and continuous escape of bubbles (two or more bubbles per second) can be observed, the heating is interrupted. It is allowed to cool slowly and the mouth of the capillary is observed. The boiling point detected is the temperature at which the flow of bubbles slows down, until it disappears, and the substance starts rising inside the capillary.

More precisely, to measure the boiling point of a substance (for example of the propellant) it is proceed as required by ASTM D86-20b standard (2020 edition).

Advantageously but not necessarily, the composition comprises up to about 80% (in particular, up to about 75%; more in particular, up to about 70%; still more in particular, up to about 65%) by weight, relative to the overall weight of the composition, of the wax.

According to specific non-limiting embodiments, the composition comprises from about 50% to about 70% (in particular, to about 65%; more in particular, to about 60%) by weight, relative to the overall weight of the composition, of the wax.

Advantageously but not necessarily, the propellant comprises at least about 10% by weight (in particular, at least about 20%; more in particular, at least about 35%), relative to the weight of the propellant, of at least one ether chosen among C₂-C₆ ethers. In other words, the aforementioned organic compound is chosen among C₂-C₆ ethers.

Advantageously but not necessarily, the organic compound is chosen among C₂-C₄ ethers (in particular, the dimethyl ether).

According to some embodiments, the propellant comprises up to about 60% by weight (in particular, up to about 50%; more in particular, about 45%), relative to the weight of the propellant, of the aforementioned organic compound chosen among C₂-C₆ ethers (in particular among C₂-C₄ ethers; more in particular, the dimethyl ether).

In particular, in these cases, the propellant may or may not also comprise one or more aliphatic compounds chosen among the C₁-C₄ aliphatic compounds.

It has been experimentally observed that the use of the ether (in particular, of the dimethyl ether) allows to obtain a better (and much faster) solubilization of wax.

Advantageously but not necessarily, the propellant comprises at least about 15% (in particular, at least about 40%; more in particular, at least about 50%) by weight, relative to the weight of the propellant, of one or more of the mentioned C₁-C₄ aliphatic compounds. In particular, in these cases, the propellant may or may not also comprise one or more ethers chosen among C₂-C₆ ethers (in particular, C₂-C₄; more in particular, dimethyl ether).

According to non-limiting embodiments, the C₁-C₄ aliphatic compounds are C₁-C₄ alkyls (in particular, the C₁-C₄ aliphatic compounds are chosen among propane, butane and a combination thereof).

In particular, in the present text by "combination" of different compounds is meant a mixture thereof.

Advantageously but not necessarily, the propellant comprises up to about 90% by weight (in particular, up to about 70%; more in particular, up to about 65%), relative to the weight of the propellant, of one or more of the C₁-C₄ aliphatic compounds (in particular, C₁-C₄ alkyls; more in particular, propane and/or butane).

According to some non-limiting embodiments, the propellant comprises from about 15% (in particular, from about 40%; more in particular, from about 50%) to about 90% (in particular, to about 70%; more in particular, to about 65%), relative to the weight of the propellant, of one or more of the C₁-C₄ aliphatic compounds (in particular, C₁-C₄ alkyls; more in particular, propane and/or butane); and from about 10% by weight (in particular, from about 20%; more in particular, from about 35%) to about 60% by weight (in particular, to about 50%; more in particular, to about 45%), relative to the weight of the propellant, of at least one ether chosen among the C₂-C₆ ethers (in particular, among the C₂-C₄ ethers; and more in particular, the dimethyl ether).

Advantageously but not necessarily, the propellant is (in particular, predominantly - more particularly, for at least 90% by weight, relative to the total weight of the propellant itself) consisting of one or more of the C₁-C₄ aliphatic compounds (in particular, C₁-C₄ alkyls; more in particular, propane and/or butane) and of at least one ether chosen among the C₂-C₆ ethers (in particular among the C₂-C₄ ethers; more in particular, the dimethyl ether).

According to some examples, the propellent comprises propane and/or butane.

According to specific but non-limiting embodiments, the propellant comprises (in particular, consists of) from about 5% (in particular, from about 20%; more in particular, from about 35%; still more in particular, from about 40%) to about 55% (in particular, to about 50%) by weight, relative to the weight of the propellant, of butane (in particular, n-butane); and/or from about 10% to about 60% (in particular, to about 40%; more in particular, to about 20%) by weight, relative to the weight of the propellant, of propane; and from about 10% (in particular, from about 25%; more in particular, from about 35%) to about 60% (in particular, to about 50%; more in particular, to about 45%) by weight, relative to the weight of the propellant, of dimethyl ether.

Advantageously but not necessarily, the composition consists (predominantly; in particular, for at least 70% by weight; more in particular, for at least 85% by weight, relative to the weight of the composition itself) of the aforementioned wax and of the aforementioned propellant.

According to some non-limiting embodiments, the aforementioned wax is a resin chosen from the group consisting of: Rosin (CAS No. 8050-09-7), glycerol ester of rosin (CAS No. 8050-31-5), glycerol ester of rosin stabilized through hydrogenation (CAS No. 65997-13-9), triethylene glycol ester of rosin (2,2'-[1,2-ethanediylbis(oxy)]bisethanol ester of rosin acids); synthetic polycyclopentadiene and hydrogenated polycyclopentadiene resins (CAS No. 68132-00-3/25568-84-7), synthetic hydrogenated styrene resins, methyl styrene resins, indene copolymers (1H-Indene, polymer with ethenylbenzene and (1-methyl ethenyl)benzene, hydrogenated) (and a combination thereof).

Advantageously but not necessarily, wax is a resin consisting predominantly (in particular, for at least about 80% by weight - more in particular, at least about 85% by weight, relative to the total weight of the resin) of abietic acid (CAS No.: 514-10-3) and/or esters thereof (in particular with glycerol) (possibly stabilised by hydrogenation).

According to some non-limiting embodiments, the resin further comprises (in particular, up to about 20% - more in particular, up to about 15%; still more in particular, up to about 10% - by weight, relative to the total weight of the resin, of) other resin acids and/or esters thereof (in particular, with glycerol).

In some cases, the resin comprises, at least 5% by weight, relative to the total weight of the resin, other resin acids and/or esters thereof (in particular, with glycerol).

In particular, the resin acids are chosen from the group consisting of: dihydroabietic acid (C₂₀H₃₂O₂ - CAS No. 19402-28-9), dehydroabietic acid (C₂₀H₂₈O₂ - CAS No. 1740-19-8), isopimaric acid (CAS No. 5835-26-7), pimaric acid (CAS No. 127-27-5) and a combination thereof.

According to specific non-limiting implementation forms, resin is chosen in the consistent group of: Rosin (CAS No.8050-09-7), rosin glycerol ester (CAS No.8050-31-5), resin acids, polycyclopentadiene (synthetic) resins (and a combination thereof). Specifically, resin includes (more specifically, it is) Rosin (CAS No.8050-09-7), rosin glyceric ester (CAS No.8050-31-5) polycyclopentadiene (synthetic) resins (and a combination thereof).

Advantageously but not necessarily, the composition comprises (in particular, from about 10% to about 15% by weight, relative to the total weight of the composition, of) an oil component (in particular, an oil or mixture of oils) chosen from the group consisting of: mineral oils (in particular white), vegetable oils (in particular, colza oil, corn oil, sunflower oil or a combination thereof) and C₁₇-C₃₉ esters (in particular, isopropyl myristate - CAS No. 110-27-0 - and/or medium chain triglycerides - with C₁₈-C₃₆ fatty acids).

According to specific embodiments, the oil component comprises (in particular consists of) mineral oils (e.g. CAS No. 8042-47-5).

Advantageously but not necessarily, the composition comprises (in particular, from about 0.1% to about 5% by weight, relative to the total weight of the composition, of) at least one plasticizer.

According to some non-limiting embodiments, the plasticizer comprises (is) an ethylene-vinyl acetate copolymer (e.g., CAS No. 108-05-4).

Advantageously but not necessarily, the plasticizer has a melting point ranging from about 80° to about 110°C (at 1 bar). Additionally or alternatively, the plasticizer has a density ranging from about 0.93 g/cm³ to about 0.97 g/cm³ (Method: ISO 1183:2019).

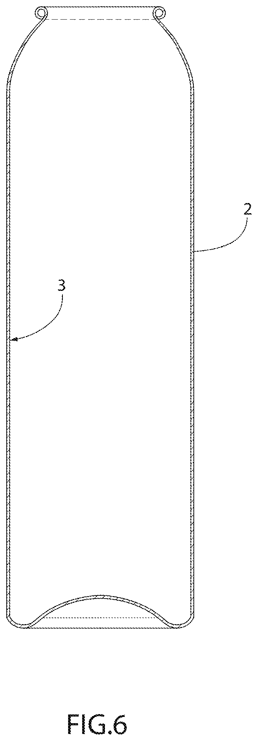

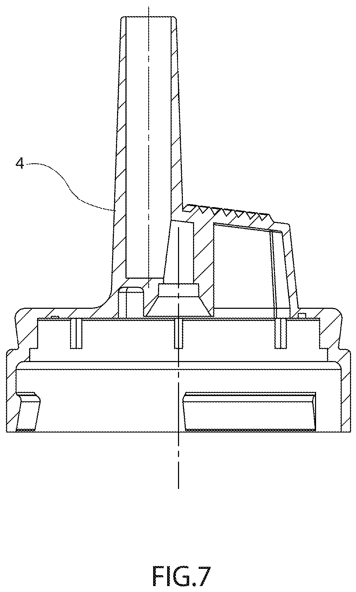

In accordance with a second aspect of the present invention, there is provided a hair removal product 1 (Figures 5-7). The product 1 is a container 2, which, in turn, is provided with a fluid-tight chamber 3 (Figure 6); a composition as described above in accordance with the first aspect of the present invention, which composition is arranged (in particular, under pressure) inside the fluid-tight chamber 3; and a dispensing device 4 (Figures 5 and 7), which is configured to dispense the composition present in the fluid-tight chamber towards the outside.

Advantageously but not necessarily, the dispensing device 4 is configured to turn the composition into foam while transferring the composition from the inside to the outside of the fluid-tight chamber 3.

Advantageously but not necessarily, the pressure inside the fluid-tight chamber 3 is between 2.5 (in particular, 2.8) and 3.5 (in particular, 3.3) bar.

In accordance with a third aspect of the present invention, there is provided a hair removal kit comprising a product 1 as described above relatively to the second aspect of the present invention and at least one sheet (in particular, a strip) of flexible material.

According to some non-limiting embodiments, the flexible material is chosen among: paper, fabric, plastic, nonwoven fabric and a combination thereof.

Advantageously but not necessarily, the kit also comprises a spatula. In particular, the spatula is configured to lay (spread) the aforementioned composition (once applied) on an area of the body.

In accordance with a fourth aspect of the present invention, there is provided a use of the composition as described above relatively to the first aspect of the present invention for the removal of hair of the human body.

Further characteristics of the present invention will become apparent from the following description of some merely illustrative and non-limiting examples.

### Example 1

This example describes the procedure for preparing a composition in accordance with the present invention.

The components used are shown in Table 1 below.

**Table 1**

| **DESCRIPTION** | **%** | **Quantity (g) Product** |
|---|---|---|
| DERCOL GL 85 (glycerol and rosin esters) | 53.878 | 323.277 |
| PHARMALUB 45 | 11 | 66.00 |
| TITANIUM DIOXIDE | 0.33 | 1.98 |
| ELVAX 250 A | 0.528 | 3.17 |
| SHEA BUTTER | 0.066 | 0.40 |
| HEMP OIL | 0.066 | 0.40 |
| SCENT | 0.132 | 0.79 |
| COLOUR CI 26100 SOL 10% IN OIL PHARMALUB 45 | 0.066 | 0.40 |
| Butane GAS 45% - Propane 15% - DME 40% | 34 | 204.00 |

DERCOL GL 85 is a wax (in particular, a resin of resin acids and rosin acids, esters with glycerol - CAS No. 8050-31-5) whose supplier is Diamantino Malho & C, Lda; PHARMALUB 45 is a white mineral oil (CAS No. 8042-47-5) whose supplier is BT Commerciale S.r.l; the supplier of titanium oxide is UNIVAR SpA; ELVAX 250 A is a plasticizer (film-forming polymer) whose supplier is Cometech (manufacturer: Dupont Performance Materials International Sàrl, 2 Chemin Du Pavilion, 1218 LE GRAND-SACONNEX, SWITZERLAND) and consists of an ethylene-vinyl acetate copolymer (N. CAS108-05-4); the supplier of SHEA BUTTER and HEMP OIL is ACEF SpA; the supplier of the SCENT is GRC Parfum SpA; the supplier of the propellant gas (combination of Butane, propane and DME - dimethyl ether) is SETTALA GAS Srl.

The following procedure was followed. In a Mixer the following components have been loaded in succession: DERCOL GL 85 (Resin), PHARMA 45 OIL (Pharmaceutical White Mineral Oil) and TITANIUM DIOXIDE.

It was heated to 120±10°C under stirring until the semi-finished product had completely melted.

Once the melting and 120±10°C were reached, the ELVAX 250A component was added and stirred until complete melting. The product was naturally cooled to 80±3°C.

Once the temperature of 80°C was reached, the remaining components (Excipients, Colour, Scent) were added to the mixture, except for the propellant gas.

The aluminium cans were filled with a cosmetic doser heated to 80±3°C and the shut-off/dispensing valves were crimped.

At this point, the propellant gas was added and the cans were capped with the appropriate dispensers.

After five days of storage it was possible to check whether the resin had adequately solubilized by shaking the cans. When the balls could be heard moving inside the can, the product was considered ready for use.

### Example 2

The functionality of the composition obtained as indicated in the previous example was tested with respect to Veet Easy-Gel^{™} depilatory strips.

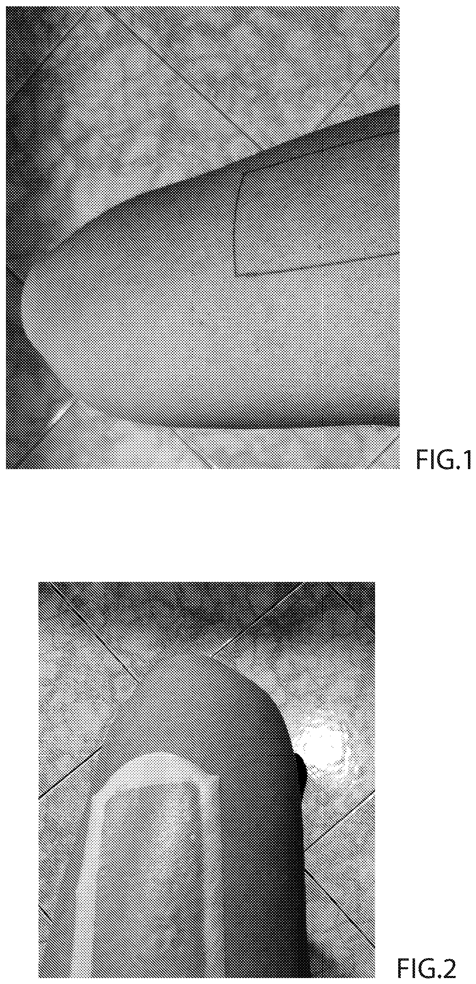

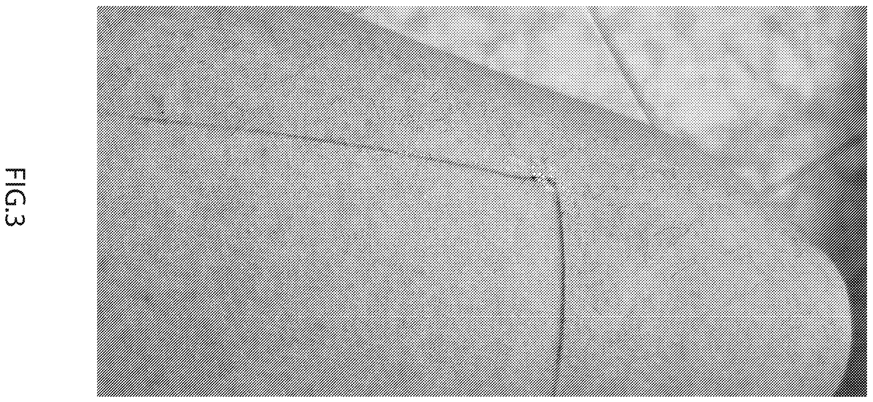

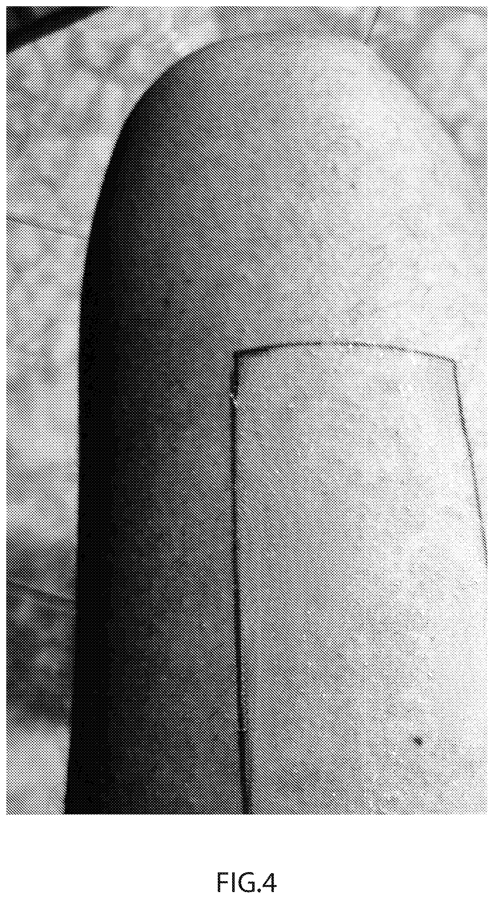

The efficacy test consisted of the epilation of two delimited areas of skin. As can be inferred from the photographs (Figures 1-4) the hair removal takes place in an excellent and substantially identical way in both cases. In this context, it should be noted that: Figure 1 shows the areas before hair removal; Figure 2 shows a Veet depilatory strip applied on the left leg and the composition obtained according to the previous example applied on the right leg; Figure 3 shows the left leg after the depilatory strip has been pulled off; and Figure 4 shows the right leg after the mentioned composition has been pulled off.

From the tests carried out, the following was observed.

The cold strips must be mechanically heated before being peeled apart, this action is drastically affected by external temperature conditions. Poor heating of the strip causes the cold strips to be peeled apart incorrectly (the wax remains only on one side of the strip and is no longer effective). Excessive heating causes excessive fluidity of the wax that remains attached to the skin without removing the hair.

By repeating the tests several times, it was therefore noted that the results obtained with the composition of the previous example are more constant. The composition has no preparatory condition and is not affected by external temperature.

### Example 3

This example describes the procedure for preparing a comparison product.

The components used are shown in Table 2 below.

**Table 2**

| **DESCRIPTION** | **%** | **Quantity (g) Product** |
|---|---|---|
| POLYSTYRENE RESIN | 8.8 | 52.80 |
| PINE RESIN | 4.4 | 26.40 |
| DIMETHYL ETHER (DME) | 43 | 258.00 |
| LPG | 43 | 258.00 |
| KAOLIN | 0.6 | 3.60 |
| PERFUME | 0.2 | 1.20 |
| **TOTAL** | **100** | **600** |

The components were sequentially loaded into a mixer: POLYSTIRENE RESIN, PINE RESIN, KAOLIN. It was heated to about 120°C under stirring until the semi-finished product was completely melted. The product was cooled naturally, and when the temperature of 80°C was reached, perfume was added.

After that, an aluminum cylinder was filled with the semi-finished product to which Propellant Gas (DME + LPG) was added. Once the closure valve was crimped, the cylinder was is closed with the appropriate nozzle.

After a few days of storage, the ball contained inside the cylinder began to move, emitting a metallic sound that defines the product as ready for use.

### Example 4

This example describes the comparison between the product obtained according to the previous example (comparison product) and the composition of Example 1.

The Evaluation Criteria follow a Reference Scale from 0 to 4, in ascending order of adequate compliance:
0 = Not applicable (NA)
1 = Not Sufficient
2 = Sufficient
3 = Good
4 = Excellent

The tests performed consisted of the application and on the skin of an area of an arm, overlapping of a depilatory strip and subsequent tearing for epilation. The results obtained are shown in Table 3 below.

**Table 3**

| **ASSESSMENT CRITERION** | **Product of example 3** | **Composition of example 1** |
|---|---|---|
| Dispensing | 1 | 4 |
| Drafting | 0 | 4 |
| Adhesion | 0 | 4 |
| Tearing | 0 | 4 |
| Effectiveness | 0 | 4 |

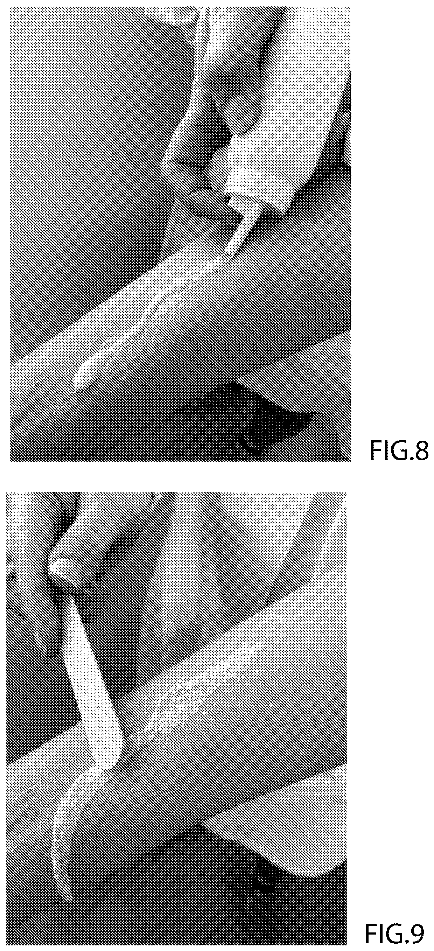

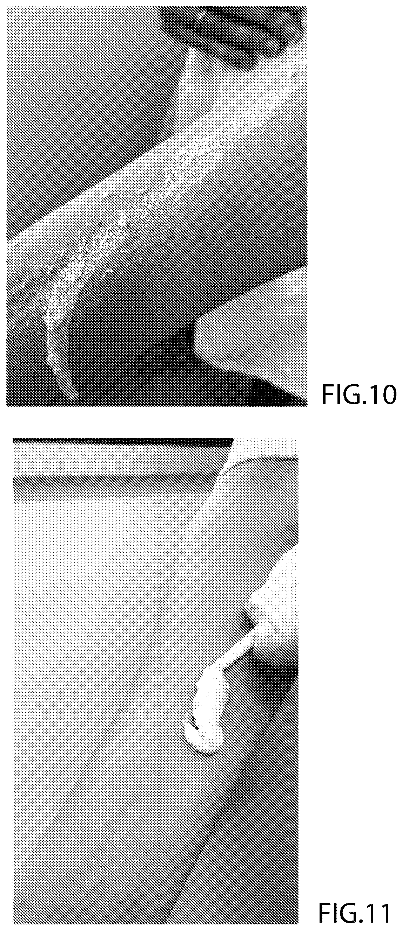

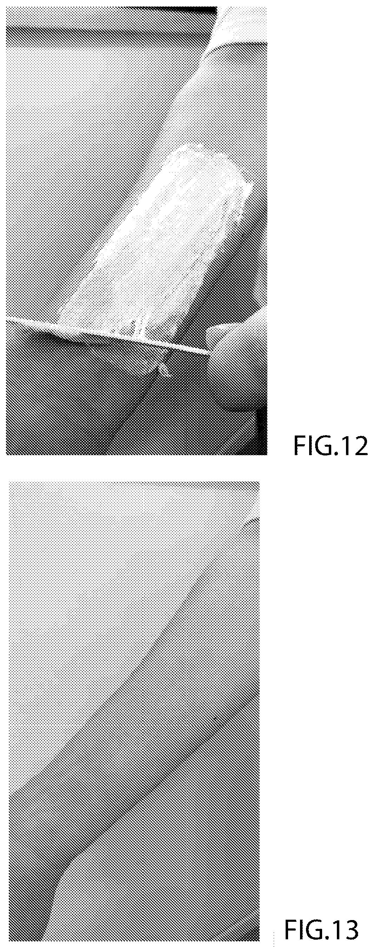

The pictures in Figures 8-10 are photographs of the tests with the (comparison) product of Example 3 while Figures 11-13 are photographs showing different stages of the tests with the composition of Example 1.

### Example 5

This example describes the preparation of additional compositions in accordance with the present invention.

The components used are shown in the following Tables 4-7. The procedure followed for the preparations is the same as described in Example 1 above.

**Table 4 (composition H313/1)**

| **DESCRIPTION** | **%** | **Quantity (g) of Product** |
|---|---|---|
| DERCOL GL-85 | 67.878 | 407.27 |
| PHARMALUB 45 | 11 | 66.00 |
| TITANIUM DIOXIDE | 0.33 | 1.98 |
| ELVAX 250 A | 0.528 | 3.17 |
| SHEA BUTTER/CHLOROPHYLL | 0.066 | 0.40 |
| PERFUME | 0.132 | 0.79 |
| COLOR | 0.066 | 0.40 |
| ***PROPELLENT GAS*** | 20 | 120.00 |
| **TOTAL** | **100** | **600** |

**Table 5 (composition H314/1)**

| **DESCRIPTION** | **%** | **Quantity (g) of Product** |
|---|---|---|
| DERCOL GL-85 | 45.878 | 275.27 |
| PHARMALUB 45 | 8 | 48.00 |
| TITANIUM DIOXIDE | 0.33 | 1.98 |
| ELVAX 250 A | 0.528 | 3.17 |
| SHEA BUTTER/CHLOROPHYLL | 0.066 | 0.40 |
| PERFUME | 0.132 | 0.79 |
| COLOR | 0.066 | 0.40 |
| ***PROPELLENT GAS*** | 45 | 270.00 |
| **TOTAL** | 100 | 600 |

**Table 6 (composition H315/1)**

| **DESCRIPTION** | **%** | **Quantity (g) of Product** |
|---|---|---|
| COLOPHONY | 53.878 | 323.27 |
| PHARMALUB 45 | 11 | 66.00 |
| TITANIUM DIOXIDE | 0.33 | 1.98 |
| ELVAX 250 A | 0.528 | 3.17 |
| SHEA BUTTER/CHLOROPHYLL | 0.066 | 0.40 |
| PERFUME | 0.132 | 0.79 |
| COLOR | 0.066 | 0.40 |
| PROPELLANT GAS | 34 | 204.00 |
| **TOTAL** | **100** | **600** |

**Table 7 (composition H316/1)**

| **DESCRIPTION** | **%** | **Quantity (g) of Product** |
|---|---|---|
| POLYCYLOPENTADIENE | 53.878 | 323.277 |
| PHARMALUB 45 | 11 | 66.00 |
| TITANIUM DIOXIDE | 0.33 | 1.98 |
| ELVAX 250 A | 0.528 | 3.17 |
| SHEA BUTTER/CHLOROPHYLL | 0.066 | 0.40 |
| PERFUME | 0.132 | 0.79 |
| COLOR | 0.066 | 0.40 |
| PROPELLANT GAS | 34 | 204.00 |
| TOTAL | **100** | **600** |

The propellant gas is: GAS Butane 45% - Propane 15% - DME 40%.

### Example 6

This example describes tests performed on the compositions in Example 5.

The evaluation criteria and procedures followed are as those given in Example 4.

The results obtained for the H313/1 and H314/1 compositions are summarized in Table 8.

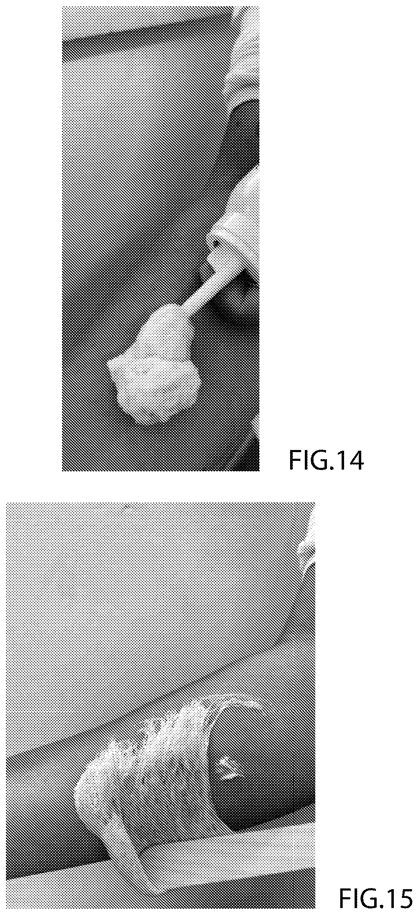

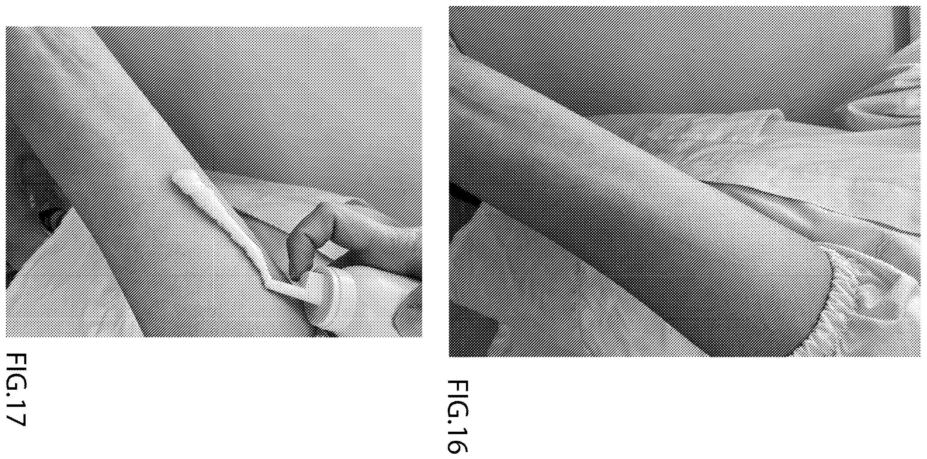

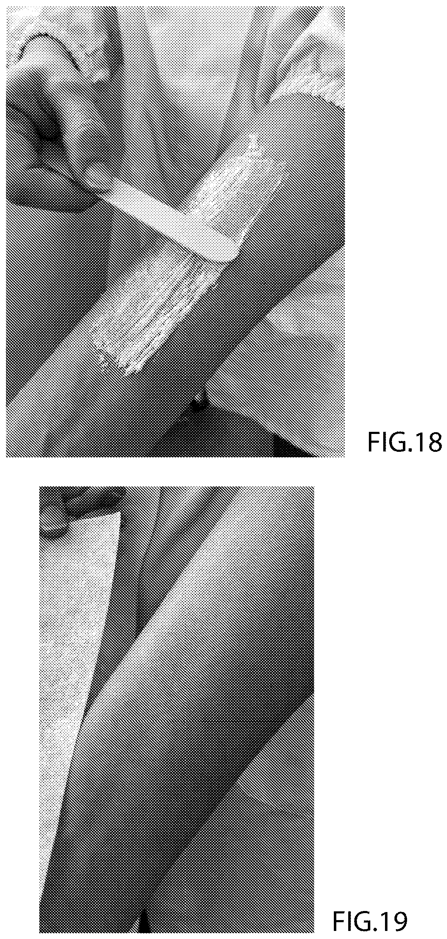

The pictures in Figures 14-16 are photographs of the tests with the H313/1 composition while Figures 17-19 are photographs showing different stages of the tests with the H314/1 composition.

**Table 8**

| **ASSESSMENT CRITERION** | **Composition H313/1** | **Composition H314/1** |
|---|---|---|
| Dispensing | 3 | 4 |
| Spreading | 3 | 4 |
| Adhesion | 4 | 4 |
| Tearing | 4 | 3 |
| Effectiveness | 4 | 3 |

Composition H313/1 is characterized by adequate delivery of the semi-finished product. The wax appears to have a more compact, apparently "harder" foaminess than the original formulation (Propellant Gas percentage of 34% by weight). This consistency, having an excellent and adequate viscosity, ensures good and even spreading. The wax adheres properly to the hair and not to the skin, allowing effective hair removal with tearing that turns out to be more firm. The result is flawless.

Composition H314/1 presents a very good delivery of the semi-finished product. The foaminess is less compact, more "liquid-like" (due to the higher percentage of gas unlike the H313/1 test) than the original formulation (Propellant Gas percentage of 34% by weight) but equally good in the acceptable ranges for excellent application. Perfect and even spreading allows good adhesion of the wax to the hair, ensuring an effective result. The soft and gentle tear allows total removal of the hair.

The results obtained for the H315/1 and H316/1 compositions are summarized in Table 9.

**Table 9**

| **ASSESSMENT CRITERION** | ***H315*/*1*** | ***H316*/*1*** |
|---|---|---|
| Dispensing | 4 | 4 |
| Spreading | 4 | 4 |
| Adhesion | 4 | 4 |
| Tearing | 4 | 4 |
| Effectiveness | 4 | 4 |

In composition H315/1, the resin used (rosin) is a natural resin, derived from the tear of the Pine tree (Gem) and subsequently purified by distillation. It has a strong and pungent characteristic odor and a fluid and very soft consistency. In the formulation, Rosin, makes effective tearing with the formation of an elastic film upon removal of the hair. With the presence of rosin in the formula, the wax does not dry out but remains soft and elastic, allowing for better spreadability and easy cold use. Its special elasticity minimizes the presence of wax residue on the skin.

The H316/1 composition with Polycyclopentadiene allows stronger and more effective tearing. The special formulation based on synthetic resin, which is highly purified and performs well in tearing, constitutes a product that respects the epidermis, giving firm and hypoallergenic hair removal. The use of the composition with Polycyclopentadiene ensures accurate and extremely effective hair removal.

The results obtained for the H315/1 and H316/1 compositions are similar to those obtained for the composition in Example 1.

Note that compositions with different types of propellants were also tested, such as: mixtures of butane and DME (55% and, respectively, 45% by weight); mixtures of propane and DME (40% and, respectively, 60% by weight); and mixtures of butane and propane (65% and, respectively, 35% by weight). In these cases, the results obtained were also good (compositions with mixtures of butane and propane only took longer to solubilize the wax).

## Claims

1. A pulling-off hair removal composition; the composition is dispensable from a spray can and comprises at least about 45% by weight, relative to the total weight of the composition, of a wax; and from about 20% to about 45% by weight, relative to the total weight of the composition, of a propellant, which is gaseous at a temperature of 25°C and at a pressure of 100000 Pa (1 bar) and liquid at a temperature of 25°C and at a pressure of 280000 Pa (2.8 bar) and comprises at least one organic compound chosen from the group consisting of C₁-C₄ aliphatic compounds, C₂-C₆ ethers and a combination thereof.

2. Composition according to claim 1, wherein the wax is a resin, more particularly wherein the resin is basically solid at a temperature below about 40°C at a pressure of 100000 Pa (1 bar).

3. The composition according to claim 1 or 2, and comprising up to about 80% (more particularly, up to about 75%) by weight, relative to the total weight of the composition, of the wax, which is substantially solid at a temperature lower than about 40°C and at a pressure of 100000 Pa (1 bar); the propellant comprises at least about 20% by weight (more particularly, at least about 25%) by weight, relative to the weight of the propellant, of said organic compound, which is chosen among C₂-C₄ ethers.

4. The composition according to claim 3, wherein said organic compound is dimethyl ether.

5. The composition according to any one of the preceding claims, wherein the wax is substantially solid at a temperature lower than about 65°C and at a pressure of 100000 Pa (1 bar) and comprises (more particularly, it is) a resin; the propellant also is gaseous at a temperature of about 0°C and at a pressure of about 100000 Pa (1 bar).

6. The composition according to any one of the preceding claims, wherein the propellant comprises at least about 15% by weight, relative to the weight of the propellant, of said C₁-C₄ aliphatic compounds; said C₁-C₄ aliphatic compounds are C₁-C₄ alkyls; more particularly, the C₁-C₄ aliphatic compounds are chosen among propane, butane and a combination thereof.

7. The composition according to any one of the preceding claims, wherein the propellant comprises from about 5% to about 55% by weight, relative to the weight of the propellant, of butane (which is more particularly n-butane); from about 10% to about 60% by weight, relative to the weight of the propellant, of propane; and from about 10% to about 60% by weight, relative to the weight of the propellant, of dimethyl ether.

8. The composition according to any one of the preceding claims, wherein said wax is a resin that is substantially liquid at a temperature of 100°C; more particularly, said resin has a melting point ranging from about 70°C to about 99°C.

9. The composition according to any one of the preceding claims, wherein said wax is a resin chosen from the group consisting of: rosin, glycerol ester of rosin, glycerol ester of rosin stabilized through hydrogenation, triethylene glycol ester of rosin, (2,2'-[1,2-ethanediylbis(oxy)]bisethanol ester of rosin acids); synthetic polycyclopentadiene and hydrogenated polycyclopentadiene resins, synthetic hydrogenated styrene resins, methyl styrene resins, indene copolymers.

10. The composition according to any one of the preceding claims, wherein said wax is a resin mainly consisting of abietic acid and/or esters of abietic acid, more particularly with glycerol.

11. The composition according to any one of the preceding claims and comprising an oil component chosen from the group consisting of: mineral oils (which, more particularly, are white oil) plant oils (which, more particularly, are colza oil, corn oil, sunflower oil and a combination thereof) and C₁₇-C₃₉ esters (which, more particularly, are isopropyl myristate and/or medium-chain triglycerides) .

12. The composition according to any one of the preceding claims and comprising at least one plasticizer, which, more particularly, is an ethylene-vinyl acetate copolymer.

13. A hair removal product; the product (1) comprises a container (2), which, in turn, is provided with a fluid-tight chamber (3); a composition according to any one of the preceding claims, said composition being arranged inside said fluid-tight chamber (3); and a dispensing device (4), which is configured to dispense said composition present in said fluid-tight chamber (3) towards the outside.

14. The product according to claim 13, wherein the dispensing device (4) is configured to turn said composition into foam while transferring the composition from the inside to the outside of said fluid-tight chamber (3); the pressure inside said fluid-tight chamber (3) ranging from 250000 to 350000 Pa (2.5 to 3.5 bar).

15. A hair removal kit comprising a product (1) according to claim 13 or 14 and at least one sheet of flexible material (more particularly, of nonwoven fabric).

16. A use of a composition according to any one of the claims from 1 to 12 for body hair removal.

## Patentansprüche

1. Haarentfernungszusammensetzung zum Abziehen; wobei die Zusammensetzung aus einer Sprühdose dosierbar ist und mindestens etwa 45 Gew.-%, relativ zum Gesamtgewicht der Zusammensetzung, eines Wachses; sowie etwa 20 bis etwa 45 Gew.-%, relativ zum Gesamtgewicht der Zusammensetzung, eines Treibmittels umfasst, das bei einer Temperatur von 25 °C und einem Druck von 100.000 Pa (1 bar) gasförmig und bei einer Temperatur von 25 °C und einem Druck von 280.000 Pa (2,8 bar) flüssig ist und mindestens eine organische Verbindung umfasst, die aus der Gruppe ausgewählt ist, die aliphatische C₁-C₄-Verbindungen, C₂-C₆-Ethern und eine Kombination davon umfasst.

2. Zusammensetzung nach Anspruch 1, wobei das Wachs ein Harz ist, insbesondere wobei das Harz bei einer Temperatur unter etwa 40 °C und einem Druck von 100.000 Pa (1 bar) im Wesentlichen fest ist.

3. Zusammensetzung nach Anspruch 1 oder 2, die bis zu etwa 80 Gew.-% (genauer gesagt bis zu etwa 75 Gew.-%) bezogen auf das Gesamtgewicht der Zusammensetzung, des Wachses umfasst, das bei einer Temperatur unter etwa 40 °C und bei einem Druck von 100.000 Pa (1 bar) im Wesentlichen fest ist; wobei das Treibmittel mindestens etwa 20 Gew.-% (insbesondere mindestens etwa 25 Gew.-%) umfasst, bezogen auf das Gewicht des Treibmittels, der organischen Verbindung, die aus C₂-C₄-Ethern ausgewählt ist.

4. Zusammensetzung nach Anspruch 3, wobei die organische Verbindung Dimethylether ist.

5. Zusammensetzung nach einem der vorstehenden Ansprüche, wobei das Wachs bei einer Temperatur unter etwa 65 °C und bei einem Druck von 100.000 Pa (1 bar) im Wesentlichen fest ist und ein Harz umfasst (genauer gesagt, es ist ein Harz); wobei das Treibmittel zudem bei einer Temperatur von etwa 0 °C und bei einem Druck von etwa 100.000 Pa (1 bar) gasförmig ist.

6. Zusammensetzung nach einem der vorstehenden Ansprüche, wobei das Treibmittel mindestens etwa 15 Gew.-%, bezogen auf das Gewicht des Treibmittels, der aliphatischen C₁-C₄-Verbindungen umfasst; wobei die aliphatischen C₁-C₄-Verbindungen C₁-C₄-Alkyle sind; genauer gesagt, die aliphatischen C₁-C₄-Verbindungen aus Propan, Butan und einer Kombination davon ausgewählt sind.

7. Zusammensetzung nach einem der vorstehenden Ansprüche, wobei das Treibmittel, bezogen auf das Gewicht des Treibmittels, etwa 5 bis etwa 55 Gew.-% Butan (insbesondere n-Butan) umfasst; etwa 10 bis etwa 60 Gew.-%, bezogen auf das Gewicht des Treibmittels, Propan; und etwa 10 bis etwa 60 Gew.-%, bezogen auf das Gewicht des Treibmittels, Dimethylether.

8. Zusammensetzung nach einem der vorstehenden Ansprüche, wobei das Wachs ein Harz ist, das bei einer Temperatur von 100 °C im Wesentlichen flüssig ist; genauer gesagt weist das Harz einen Schmelzpunkt im Bereich von etwa 70 °C bis etwa 99 °C auf.

9. Zusammensetzung nach einem der vorstehenden Ansprüche, wobei das Wachs ein Harz ist, das aus der Gruppe ausgewählt ist, bestehend aus: Kolophonium, Glycerinester von Kolophonium, durch Hydrierung stabilisierter Glycerinester von Kolophonium, Triethylenglykolester von Kolophonium, (2,2'-[1,2-Ethandiylbis(oxy)]bisethanolester von Kolophoniumsäuren); synthetischen Polycyclopentadien- und hydrierten Polycyclopentadienharze, synthetischen hydrierten Styrolharzen, Methylstyrolharzen, Inden-Copolymeren.

10. Zusammensetzung nach einem der vorstehenden Ansprüche, wobei das Wachs ein Harz ist, das hauptsächlich aus Abietinsäure und/oder Estern der Abietinsäure, insbesondere mit Glycerin, besteht.

11. Die Zusammensetzung nach einem der vorstehenden Ansprüche, die eine Ölkomponente umfasst, die aus der Gruppe ausgewählt ist, bestehend aus: Mineralölen (insbesondere Weißöl), Pflanzenölen (insbesondere Rapsöl, Maisöl, Sonnenblumenöl und einer Kombination davon) sowie C₁₇-C₃₉-Estern (insbesondere Isopropylmyristat und/oder mittelkettigen Triglyceriden).

12. Zusammensetzung nach einem der vorstehenden Ansprüche, die mindestens einen Weichmacher umfasst, bei dem es sich insbesondere um ein EthylenVinylacetat-Copolymer handelt.

13. Haarentfernungsprodukt; wobei das Produkt (1) einen Behälter (2) umfasst, der seinerseits mit einer flüssigkeitsdichten Kammer (3) bereitgestellt ist; eine Zusammensetzung nach einem der vorstehenden Ansprüche, wobei die Zusammensetzung innerhalb der flüssigkeitsdichten Kammer (3) angeordnet ist; und eine Abgabevorrichtung (4), die so konfiguriert ist, dass sie die in der flüssigkeitsdichten Kammer (3) vorhandene Zusammensetzung nach außen abgibt.

14. Produkt nach Anspruch 13, wobei die Abgabevorrichtung (4) konfiguriert ist, dass sie die Zusammensetzung in Schaum umwandelt, während sie die Zusammensetzung von der Innenseite zur Außenseite der flüssigkeitsdichten Kammer (3) befördert; wobei der Druck innerhalb der flüssigkeitsdichten Kammer (3) im Bereich von 250.000 bis 350.000 Pa (2,5 bis 3,5 bar) liegt.

15. Haarentfernungsset, umfassend ein Produkt (1) nach Anspruch 13 oder 14 und mindestens eine Folie aus flexiblem Material (insbesondere aus Vliesstoff)

16. Verwendung einer Zusammensetzung nach einem der Ansprüche 1 bis 12 zur Entfernung von Körperbehaarung.

## Revendications

1. Composition d'épilation par arrachage ; ladite composition pouvant être distribuée à partir d'un aérosol et comprenant au moins environ 45 % en poids, par rapport au poids total de la composition, d'une cire ; et d'environ 20 à environ 45 % en poids, par rapport au poids total de la composition, d'un gaz propulseur, qui est gazeux à une température de 25 °C et à une pression de 100 000 Pa (1 bar) et liquide à une température de 25 °C et à une pression de 280 000 Pa (2,8 bars), et qui comprend au moins un composé organique choisi dans le groupe constitué par les composés aliphatiques en C₁-C₄, les éthers en C₂-C₆ et une combinaison de ceux-ci.

2. Composition selon la revendication 1, la cire étant une résine, plus particulièrement la résine étant essentiellement solide à une température inférieure à environ 40 °C à une pression de 100 000 Pa (1 bar).

3. Composition selon la revendication 1 ou 2, et comprenant jusqu'à environ 80 % (plus particulièrement, jusqu'à environ 75 %) en poids, par rapport au poids total de la composition, de cire, qui est sensiblement solide à une température inférieure à environ 40 °C et à une pression de 100 000 Pa (1 bar) ; le gaz propulseur comprend au moins environ 20 % en poids (plus particulièrement, au moins environ 25 %) en poids, par rapport au poids du gaz propulseur, dudit composé organique, qui est choisi parmi les éthers en C₂-C₄.

4. Composition selon la revendication 3, ledit composé organique étant le diméthyléther.

5. Composition selon l'une quelconque des revendications précédentes, la cire étant sensiblement solide à une température inférieure à environ 65 °C et à une pression de 100 000 Pa (1 bar) et comprend (plus particulièrement, étant) une résine ; le gaz propulseur étant également à l'état gazeux à une température d'environ 0 °C et à une pression d'environ 100 000 Pa (1 bar).

6. Composition selon l'une quelconque des revendications précédentes, le gaz propulseur comprenant au moins environ 15 % en poids, par rapport au poids du gaz propulseur, desdits composés aliphatiques en C₁-C₄ ; lesdits composés aliphatiques en C₁-C₄ étant des groupes alkyle en C₁-C₄ ; plus particulièrement, les composés aliphatiques en C₁-C₄ étant choisis parmi le propane, le butane et une combinaison de ceux-ci.

7. Composition selon l'une quelconque des revendications précédentes, le gaz propulseur comprenant, par rapport au poids du propulseur, environ 5 à environ 55 % en poids de butane (étant plus particulièrement du n-butane) ; d'environ 10 à environ 60 % en poids, par rapport au poids du gaz propulseur, de propane ; et d'environ 10 à environ 60 % en poids, par rapport au poids du gaz propulseur, de diméthyléther.

8. Composition selon l'une quelconque des revendications précédentes, ladite cire étant une résine qui est sensiblement liquide à une température de 100 °C ; plus particulièrement, ladite résine présentant un point de fusion compris entre environ 70 et environ 99 °C.

9. Composition selon l'une quelconque des revendications précédentes, ladite cire étant une résine choisie dans le groupe constitué par la colophane, l'ester glycérinique de colophane, l'ester glycérinique de colophane stabilisé par hydrogénation, l'ester de triéthylèneglycol de colophane, (l'ester 2,2'-[1,2-éthanediylebis(oxy)]biséthanol des acides de colophane) ; des résines de polycyclopentadiène synthétique et de polycyclopentadiène hydrogéné, des résines de styrène synthétique hydrogéné, des résines de méthylstyrène, des copolymères d'indène.

10. Composition selon l'une quelconque des revendications précédentes, ladite cire étant une résine constituée principalement d'acide abiétique et/ou d'esters d'acide abiétique, plus particulièrement avec du glycérol.

11. Composition selon l'une quelconque des revendications précédentes, comprenant un composant huileux choisi dans le groupe constitué par des huiles minérales (qui sont, plus particulièrement, de l'huile blanche), des huiles végétales (qui sont, plus particulièrement, de l'huile de colza, de l'huile de maïs, de l'huile de tournesol et d'une combinaison de celles-ci) et d'esters en C₁₇-C₃₉ (qui sont, plus particulièrement, le myristate d'isopropyle et/ou des triglycérides à chaîne moyenne).

12. Composition selon l'une quelconque des revendications précédentes et comprenant au moins un plastifiant, qui est, plus particulièrement, un copolymère d'éthylène-acétate de vinyle.

13. Produit d'épilation ; le produit (1) comprend un récipient (2) qui, à son tour, est pourvu d'une chambre étanche aux fluides (3) ; composition selon l'une quelconque des revendications précédentes, ladite composition étant disposée à l'intérieur de ladite chambre étanche aux fluides (3) ; et un dispositif de distribution (4), qui est configuré pour distribuer ladite composition présente dans ladite chambre étanche aux fluides (3) vers l'extérieur.

14. Produit selon la revendication 13, le dispositif de distribution (4) étant conçu pour transformer ladite composition en mousse tout en transférant celle-ci de l'intérieur vers l'extérieur de ladite chambre étanche aux fluides (3) ; la pression à l'intérieur de ladite chambre étanche aux fluides (3) étant comprise entre 250 000 et 350 000 Pa (2,5 à 3,5 bars).

15. Kit d'épilation comprenant un produit (1) selon la revendication 13 ou 14 et au moins une feuille de matériau souple (plus particulièrement, de tissu non tissé)

16. Utilisation d'une composition selon l'une quelconque des revendications 1 à 12 pour l'épilation des poils du corps.
